# EUROPEAN PATENT APPLICATION

(11) **EP 1 378 520 A1**
(43) Date of publication of application: **07.01.2004**
(21) Application number: 02724089.4
(22) Date of filing: 10.04.2002
(51) Int. Cl.: C07K 16/18, A61K 39/395, C12N 15/13, A61P 35/00

(54) **CYCLIC SINGLE STRAND TRISPECIFIC ANTIBODY**

(30) Priority: 11.04.2001 CN 01110554
(71) Applicant: Institute of Genetics, Chinese Academy of Sciences, Beijing 100101 (CN); Beijing ABT Genetic Engineering Technology Co., Ltd., Beijing 100085 (CN); Dongguang Haofa Biotechnology Developmental Co. Ltd, Dongguan, Guangdong 523980 (CN)
(72) Inventor: HUANG, Hua-Liang, Beijing 100101 (CN); CHENG, Ju-Long, Beijing 100101 (CN); WANG, Xiang-Bin, Beijing 100101 (CN); SONG, Li-Ping, Beijing 100085 (CN); ZHANG, Zhong, Beijing 100085 (CN); LIN, Qing, Beijing 100085 (CN); GU, Ying, Beijing 100085 (CN)
(74) Representative: Viering, Jentschura & Partner
(86) International application number: PCT/CN2002/000252
(87) International publication number: WO 2002/083738

(57) **Abstract**

The invention provides a cyclic single-chain trispecific antibody against human tumor. It comprises three parts. The first part is an anti-tumor Fab antibody, an anti-tumor single-domain antibody or an scFv. The second part is a reshaped Fab antibody against human CD3, a reshaped single-domain antibody against human CD3 or a reshaped scFv against human CD3. The third part is a reshaped Fab antibody against human CD28, a reshaped single-domain antibody against human CD28 or a reshaped scFv against human CD28. The present invention also offers the DNA sequence coding for this trispecific antibody, expression vectors containing this DNA sequence and host cells *(E. coli)* containing the vectors.

## Description

### BACKGROUND OF THE INVENTION

### 1.FIELD OF THE INVENTION

The present invention relates to an engineered cyclic single-chain trispecific antibody, DNA sequences coding it, expression vectors containing the said sequences as well as host cells containing the said expressing vectors.

### 2. DESCRIPTION OF THE RELATED ART

Strategy of constructing trispecific antibody is based on introducing three different antigen-binding sites into a single molecule. Since the antibody genes selected for construction are different, so the trispecific antibody has various biological functions. The trispecific antibodies reported were mainly constructed by chemical coupling method, hybrid hybridoma technique or genetic fusion expression, these antibodies contained three Fab fragments or only one single-chain antibody (scFv) among three antibodies *( Fay TN* et *al, 1988; Tutt A et al, 1991; Jung G et al, 1991; Schott ME et al, 1993; French RR, 1998; Somasundaram C et al, 1999; Schoonjans R et al, 2000a; Schoonjans R et al, 2000b; Wong WM et al, 2000).*

The cyclic single-chain trispecific antibody mentioned in this invention is a new kind of engineering antibody, designed based on the model of two signals activation for T cell that play a key function in immunotherapy of cancer. In tumor immunotherapy T cell-mediated cell immunity plays a key role. Full activation of T cells requires two signals: the primary signal is provided by the TCR/CD3 complex; which is related with antigen specificity; the second signal, also called co-stimulatory signal, is afforded by the co-stimulatory molecules on the surface of APC. Human CD3 consists of five different polypeptide chains. CD3 and TCR constitute the CD3/TCR complex with non-covalent bonds. If T cells receive only primary signal (TCR *binding*), without co-stimulatory signal, T cells will be induced anergy, or apoptosis. The co-stimulatory signal is non-antigen specificity and non-MHC restriction but involve in inducing the secretion of cytokines, the proliferation and effect function of T cells. CD28 is the most important receptor of co-stimulatory signal on the surface of T cells. Among various receptors of co-stimulatory signal on T cell, such as CD2, CD4, CD8 etc, only CD28 can prevents the induction of T cell anergy *(Slavik et al, 1999).* Based on these principles, T cell can be activated by using anti-CD3 antibody and anti-CD28 antibody as the ligands of these molecules respectively.

The development of engineering antibody technology especially the application of genetic engineering technology in antibody modification has facilitated the alteration of antibody according to the need in application. In recent years, some bispecific antibodies and bispecific single-chain antibodies with different targeting properties have been produced, which can recognizes cancer cells and direct to stimulate immune effector cells.

Among the reports about genetic engineered antibodies in tumor immunotherapy, most of them were related to bispecific antibodies *(BsAb)* or mAbs with specificity to tumor-associated antigen *(TAA)* and CD3 or CD28. In the earlier clinical experiments for tumor immunotherapy, BsAb was produced by coupling two antibodies against trigger molecule TCR-CD3 and TAA. But the therapy effect was disappointed since it could lead to the activated T cell clone anergy and apoptosis. This problem might be circumvented by *ex vivo* activation of T cell through using IL-2 or lectin as co-stimulatory molecule. With more understanding of CD28 molecule and development of double stimulatory signal theory, anti-CD28 mAb was found to be able to deliver co-stimulatory signal as the B7 family, and cooperate with anti-CD3/TAA to trigger optimal activation of T cell effectively.

Demanet *et al (1996)* demonstrated that the lymphoma loaded 10⁵ BCL1 cells was eliminated when anti-CD3/anti-Id BsAb plus anti-CD28 Mab were injected into Balb/C mice model for several times. Comparing with single application of BsAb, the curative effect was increased by 20 fold, while the dose of BsAb was only 10 percent of that of single BsAb. Using the SCID mice model with human chronic B lymphocyte leukemia. Bohlen *et al (1997)* demonstrated that combined injection of anti-CD3/anti-CD19 BsAb with anti-CD28 McAb into the mice could mediate the autologous T cells to inhibit the growth of tumor cells and prevent recrudescence of the tumor. In further research, anti-CD3/anti-TAA BsAb and anti-CD28/anti-TAA BsAb were used together to improve the specificity to tumor cells. For example, Renner *et al(1994)* reported that combined injection of anti-CD3/anti-CD30 BsAb with anti-CD28/anti-CD30 BsAb into SCID mice with human Hodgkin's disease produced exciting killing effect. Mazzoni *et al (1996)* simultaneously applied anti-CD3/anti-FBP *(ovary carcinoma TAA)*BsAb and anti-CD28/anti-FBP BsAb in killing assay *in vitro*, the results showed that the double-signals can activate CD8⁺ T cells effectively to kill the ovary carcinoma cells with FBP antigen specifically. Comparing the results using anti-CD3/anti-CD19 BsAb only, Manzke *et al (1999)* achieved promising effect when using anti-CD3/anti-CD19 BsAb and bivalent anti-CD28 antibody simultaneously in treating B cell mediated lymphocyte cancer. It was also obtained significant effect in treatment of solid tumor with BsAb. Using a mice model with transplanted B16 melanoma and lung cancer, Grosse-Hovest *et al (1999)* showed that the anti-CD3/anti-tumor BsAb had a significant treating effect, but it could be remarkably enhanced the killing-rate of tumor cells by synchronously injecting anti-CD28 BsAb with anti-CD3/anti-tumor BsAb. Moreover, when attacked with tumor cells again, the amount of long-term survivors was significantly increased in the mice treated by i.v. injection of BsAb, which proved that BsAb could induce long-term protective immunity.

Thus, if the genes of anti-TAA antibody, anti-CD3 antibody and anti-CD28 antibody are linked together and expressed by genetic engineering technology, the resulted antibody will activate effector cells and cure tumor with higher efficiency. At the same time, the whole productive procedure will greatly become simple, efficient, lower cost. However, the antibody would be unstable and difficult to transport *in vivo* if the three antibodies just linked one by one to form a linear molecule. To solve this problem, the present invention will establish a method to construct cyclic single-chain trispecific antibody (*TsAb*) by cyclizing the linear antibody molecule with fragments of antibody hinge region.

In addition, there are many problems that need to solve in murine mAb when it used in clinic therapy. One major problem is HAMA (human anti-mouse antibody) response in patient resulting from immunogenicity of mouse-derived mAb. The HAMA response makes the mouse antibodies rapid clearance from the blood, neutralizes and blocks the function of antibody, causes the patient to have an allergic reaction. Because it is very difficult to make human monoclonal antibody with hybridoma technology, it will be a selective way to fully apply the rodent monoclonal antibodies by humanizing modification of the antibodies with genetic and protein engineering techniques.

The strategies of antibody humanization are based on the knowledge about distinguishable structure and distinct domain of antibody. Antibody is shaped like a capital letter "Y" and consists of two identical long "heavy" chains and two identical short "light" chains. Each chain has one variable domain and one or several constant domain. The variable domains are mainly responsible for the binding to the antigen while the constant domains are responsible for binding the effector molecules. The variable domains contain three flexible loop regions, which is hypervariable in sequence and crystal structure and binds antigens directly, termed the complementarity determining regions *(CDRs).* The rest of variable domains shows less variability and consist of antiparallel beta-sheets and are called framework regions *(FRs).* CDRs and FRs array one by one to form a sandwich structure. Between the heavy- and light-chains or between two heavy-chains, it was linked by disulfide bond. The conservative features of antibody structure enable it to be modified by genetic engineering and protein engineering techniques to keep its antigen-binding specificity and effector function, at the same time reduce its immunogenicity with maximum possibility for application clinical therapy.

The first generation of humanized antibody is human-mouse chimeric antibody, which consists of rodent variable regions and human constant regions. It has been demonstrated that chimaric antibody could significantly enhance pharmacodynamics coefficient and decrease immunogenicity, some of them had been applied in clinical trial. However, the result of clinic trial showed that more than half of the patients treated with chimeric antibodies generated HAMA response after repeated injection. The second generation of humanized antibody was called "CDR-grafted" antibody, in which rodent CDRs were transplanted into human FRs. Comparing to the chimaric antibody it is further humanized to make the antibody more human-like while it keeps the antigen-binding specificity of rodent antibody. In principle, different murine CDRs can be transplanted into the same human FRs and produce various reshaped antibodies with different sequences. However, this over-simple graft usually produces antibodies with poor even no activity since additional alterations of individual amino acid residues within the framework may be effect on the conformation of antigen-binding site. Thus it is invariably necessary to consider possible interactions between the amino acid residues of FRs and CDRs. The murine individual residues that served to hold the CDRs in their correct spatial construction for antigen-binding should be retained. For example, when only the CDRs of rodent antibody against human lymphocyte surface antigen were grafted into human framework, the affinity of the resulted reshaped antibody was unacceptable. Using computer to model the VH CDRs and FRs, it was found that Phe27 in FR1 contacted closely with CDR1 in rodent antibody but Ser27 in human antibody. When the serine residue at position 27 had been replaced with phenylalanine, the reshaped antibody kept the original binding activity of rodent antibody. In fact the affinity of some reshaped antibody can be improved more than 3 folds after mutation of several individual FR residues. The first reshaped antibody, CAMPATH-1H, has been used successfully in the clinic therapy of non-Hodgkin lymphoma and rheumatoid arthritis. Similar results are also obtained from HuRSV-19 and D1.3VHFNS/VK.

The general strategy of residue replacement involves selecting the most homologous human sequence as acceptor FRs, referring to the known crystal structure of variable regions and the sequences of corresponding families, building the molecular model with computer, and then determining which residue to be replaced. However, It is noticeable that adjusting the key FR residues may increase the heterogeneity of antibody, as well as the affinity of it. So it is necessary to optimize repeatedly the balance between the affinity and the immunogenicity in constructing a good therapeutic reshaped antibody. In this invention, we construct the cyclic trispecific antibody with reshaped anti-CD3 scFv and reshaped anti-CD28 single-domain antibody, which were constructed in our own library. It will be helpful to reduce the immunogenicity of the whole molecule and for further clinical therapy.

In the construction of cyclic trispecific antibody, selection of interlinker is very important because interlinker will determine whether the construction would be successful as desire. In this invention, we choose a part of Fc fragment of human IgG, a part of human serum albumin and the hinge region of human IgG3' CL as the interlinkers. A flexible short peptide *(Gly*_{*4*}*Ser)* is used between the interlinkers and antibody fragments to facilitate the different antibody fragments to fold individually and correctly.

Interlinker Fc: Unable to activate effector cascade due to the absence of Fc is the main shortcoming of small molecular antibodies. Among the four subclass of human IgG, it has been proved that IgG1 can mediate ADCC and CDC effect most efficiently. Some C-terminal residues of IgG1 CH2 can bind with C1q to trigger classical complement pathway. Of those residues, Glu318, Lys320 and Lys322 are closed in spatial and locate on the surface of Fc to bind to C1q directly. Some studies also showed that glycosylation of Fc at Asn297 was very important for ADCC and CDC without any influence to the antigen-binding activity of antibodies. Hence, a 26 amino acid fragment of human IgG1 CH2 from Asn 297 to Lys322 *(including glycosylation site and C1q binding site*) is chosen as an interlinker in the construction of trispecific antibody in the invention.

Interlinker HAS: The other problem of small molecule antibody in clinic practice is its short half-life and rapid clearance from blood, which is a vital defect for immunotherapy, although it is advantage for immunodiagnosis and neutralization of toxin. Human serum albumin is a major serum protein and spreads widely in human body. Without any enzyme activity, immune activity and side-effect, HSA is removed slowly via liver and exists in vivo for several weeks. It has been demonstrated that the stability of protein linked to HSA be increased by 20-40 folds and the fusion protein was mainly uptaken by liver for clearance, which reduce the toxicity to the kidney remarkably. HSA molecule is made up of three domains that contain 585 amino acid residues and 17 disulfide bonds. Domain III has been verified that it can function as the intact HSA protein. As a result, a fragment of 25 amino acid residues from 403 to 425 of domain III is chosen as interlinker in the invention.

Human IgG3' CL hinge: Cysteine in hinge region can form disulfide bond to facilitate the conjunction between two heavy chains in the formation of antibody with natural spatial structure. Human IgG3' CL hinge region composes of 17 amino acid residue including two cysteines, and this makes it suitable to act as interlinker. In the invention a fragment of 17 amino acid residues in hinge region of human IgG3'CL is utilized to cyclize the trispecific antibody.

*[References: 1. Huang HL. Gene engineering antibody. Monoclonal Antibody Communication, 1991, 7(3): 1-4; 2. Liu XF, Huang HL. Progress in gene engineering antibody. Progress Biotechnol, 1994, 14(1): 54; 3. Huang HL. Humanized antibody: small molecule antibody and tumor therapy. Monoclonal Antibody Communication, 1993, 9(3): 19; 4. Slavik,J.M.,Hutchcroft,J.E.&Bierer,B.E.(1999): CD28*/*CTLA-4 and CD80*/*CD86 families,signaling and function. Immunologic Research. 19*/*1:1-24; 5. Demanet C, Brissinck J, Leo O et al. : Bispecific antiboddy-mediated immunotherapy of the BCL1 lymphoma:increasd efficacy with multiple injections and CD28-induced costimulation. Blood 1996; 87: 4390-4398; 6. Bohlen H, Manzke O , Titzer S et al.: Prevention of Epstein-Barr virus-induced human B-cell lymphoma in severe combined immunodeficient mice treated with CD3 × CD19 bispecific antibodies,CD28 monospecific antibodies, and autologous T cells. Cancer Res. 1997,- 57: 1704-1709; 7. Renner C, Jang W,Sahin U et al. Science 1994; 264:833-835;8. Mazzoni A, Mezzanzanica D, Jung G et al.: CD3-CD28 costimulation as a means to avoiding T cell preactivation in bispecific monoclonal antibody-based treatment of ovarian carcinoma. Cancer Res. 1996;56:5443-5449;9. Manzke, O., Berthold, F, Huebe, K. et* *al.(1999): CD3×Cd19 bispecific antibodies and CD28 bivalent antibodies enhance T-cell reactivity against autologous leukemic cells in pediatric B-All bone marrow. Int. J. Cancer, 80:715-722;10. Grosse-Hovest L,Brandl M, Dohlsten M et al. : Int. J. Cancer 1999;80:138-144;11. Boulianne,GL.,Hozumi,N.&Shulman,M.J.(1984): Production of functional chimeric mouse*/*human antibody. Nature. 312, 643-646; 12. Neuberger,M.S., Williams,GT. & Fox,R.O. (1984): Recombinant antibodies possessing novel effector functions. Nature 312,604-608;13. Jones, P.T.,Dear,P.H., Foote,J. et al. (1986): Replacing the complementarity-determining regions in a human antibody with those from a mouse. Nature, 321,522-525; 14. Riechmann,L., Clark, M., Waldmann, H. et al. (1988): Reshaping human antibodies for therapy. Nature,332,323-327; 15. Fay TN, Jacobs I, Teisner B. et al.(1988): Two fetal antigens (FA-1 and FA-2) and endometrial proteins (PP12 and PP14) isolated from amniotic fluid; preliminary observations in fetal and maternal tissues. Eur J Obstet Gynecol Reprod Biol ,29(1):73-85; 16. Tutt A, Stevenson GT, Glennie MJ(1991): Trispecific F(ab')3 derivatives that use cooperative signaling via the TCR*/*CD3 complex and CD2 to activate and redirect resting cytotoxic T cells. J Immunol 147(1):60-9; 17. Jung G, Freimann U, Von Marschall Z, et al.(1991): Target cell-induced T cell activation with bi- and trispecific antibody fragments. Eur J Immunol 21(10):2431-5: 18. French RR,(1998): Production of bispecific and trispecific F(ab)2 and F(ab)3 antibody derivatives. Methods Mol Biol, 80:121-134; 19. Somasundaram C, Sundarapandiyan K, Keler T, et al., (1999): Development of a trispecific antibody conjugate that directs two distinct tumor-associated antigens to CD64 on myeloid effector cells. Hum Antibodies, 9(1):47-54; 20. Schoonjans R, Willems A, Schoonooghe S, et al.(2000a): Fab chains As an efficient heterodimerization scaffold for the production of recombinant bispecific and trispecific antibody derivatives. J Immunol ,165(12):7050-7; 21. Schoonjans R, Willems A, Grooten J, et al.,(2000b): Efficient heterodimerization of recombinant bi- and trispecific antibodies. Bioseparation ,9(3):179-83; 22. Wong WM, Vakis SA, Ayre KR, et al.,(2000): Rheumatoid arthritis T cells produce Thl cytokines in response to stimulation with a novel trispecific antibody directed against CD2, CD3, and CD28. Scand J* *Rheumatol ,29(5):282-7; 23. Schott ME, Frazier KA, Pollock DK,et al.,(1993): Preparation, characterization, and in vivo biodistribution properties of synthetically cross-linked multivalent antitumor antibody fragments. Bioconjug Chem ,4(2):153-65]*

The incidence of ovarian carcinoma is the second in gynecologic malignancy. The most serious nodus of this disease is absence of symptoms in early-stage, prone to recurrence and rather low five-year livability (30%). To improve its post-cure situations, it is critical to develop a sensitive early diagnostic method and an effective approach to eliminate the remained focus after surgical operation. In this way, the cyclic single-chain trispecific antibody will be helpful during the immunotherapy of ovarian carcinoma.

### SUMMARY OF THE INVENTION

The object of the present invention is to provide a specifically designed engineering anti-tumor×reshaped anti-CD3×reshaped anti-CD28 cyclic single-chain trispecific antibody with low toxicity, high efficiency and simplified techniques to produce.

In a preferred embodiment, the present invention provides an expression vector which can be used in constructing a universal cyclic single-chain trispecific antibody.

In another aspect, the present invention provides a host cell containing the expression vector used in constructing cyclic single-chain trispecific antibody.

In yet another aspect, the present invention provides a nucleotide sequence coding for that said cyclic single-chain trispecific antibody.

An antibody molecule comprises two identical pairs of heavy chains and light chains. Each of chains is composed of one variable region (V) and one or more constant region (C). The V regions are responsible for antigen binding and C regions for effector molecule binding. Within every variable regions, there are three short flexible loop segments, which are entitled as complementarity-determining regions *(CDRs)* and variable in sequence and crystal structure, while the other intervening segments known as framework regions *(FRs)* are relative stable, and is composed of β -sheet. These CDRs and FRs arrange at intervals and form a "sandwich" structure. The terms used in present invention are list as follows.

"Fab antibody" is a fragment of antibody containing Fd fragment *(V*_{*H*} *of heavy chain +CH1*) and entire light chain. They form a hetero-dimer by disulfide bond. It is about 1/3 of an entire antibody molecule in size and has only one antigen-binding site.

"Single-chain antibody (scFv)" is a recombinant protein produced by genetic engineering technology. It is composed of a VH and a VL connected with a linker peptide. It is about 1/6 of an entire antibody molecule in size.

"Single-domain antibody" is referred to a variable region of heavy chain or light chain. This type of engineering antibody fragment has only one domain and is about 1/12 of an entire antibody in size.

"Minimal recognizing unit (MRU)" is any single CDR of variable regions of heavy chain or light chain. It is about 1/70~1/80 of an entire antibody molecule in size.

In "reshaped antibody" (also known as CDR-grafted antibody), the substitution of murine CDRs for human CDRs is carried out by artificial synthesis or site-directed mutagenesis, so it remains the antigen-binding activity of original murine monoclonal antibody. Some amino acid residues in human FRs may interfere with the conformation of antigen-binding site, so these amino acids have to be altered to get a highest affinity humanized antibody to the greatest extent.

The present invention provides a cyclic single-chain trispecific antibody against tumor. It comprised of three parts: an anti-tumor Fab, single-domain antibody or scFv, a reshaped Fab, single-domain antibody or scFv against human CD3 molecule, and a reshaped Fab, single-domain antibody or scFv against human CD28 molecule, they are ligated by some interlinker peptides to form a cyclic single-chain molecule.

The anti-tumor antibody of the cyclic single-chain trispecific antibody mentioned in this invention may be a Fab fragment, a single-domain antibody or a single-chain antibody against human ovarian carcinoma.

It is the best that the cyclic single-chain trispecific antibody is composed of a single-chain antibody against carcinoma, a reshaped single-chain antibody against human CD3 and a reshaped single-domain antibody against human CD28, which are ligated by some interlinker peptides to form a cyclic single-chain molecule.

It is better that the single-domain antibody of the cyclic single-chain trispecific antibody mentioned in this invention is the V_{H} of antibody against CD28, whose amino acid sequence is one of following sequences:

Q V Q L Q E S G P G L V K P S Q T L S L T C T V S G F S L S D Y G V H W V R Q
P P G K G L E W L G V I W G G G T N Y N S A L M S R R V T S S D D T S K N Q
F S L K L S S V D T A V Y Y C A R S Y Y Y S M D Y W G Q G T L V T V S S
(113aa)or

QVQLQESGPGLVKPSQTLSLTCTVSGFSLSDYGVHWVRQ
PPGKGLEWLGVIWAGGGTNYNSALMSRRVTSSDDTSKNQ
FSLKLSLSSVDTAVYYCARDKGYSYYYSMDYWGQGTLVTVSS
(126aa)

In the cyclic single-chain trispecific antibody mentioned in this invention, there has better been some kinds of interlinker peptides between the anti-tumor antibody (Fab, single-domain antibody or scFv), reshaped CD3 antibody (Fab, single-domain antibody or scFv) and reshaped CD28 antibody (Fab, single-domain antibody or scFv). That said interlinker peptides may has one of following amino acid sequences:

(1) pelB

1 ATGAAATACCTATTGCCTACGGCAGCCGCTGGATTGTTATTACTCGCTGCCCAACCAGCC
TACTTTATGGATAACGGATGCCGTCGGCGACCTAACAATAATGAGCGACGGGTTGGTCGG
1 M K Y L L P T A A A G L L L L A A Q P A

61 ATGGCCCAGGTGAAACTG
TACCGGGTCCACTTTGAC
21 M A Q V K L

(2)Gly4Ser
1   GGTGGTGGTGGTTCT
    CCACCACCACCACGC
1    G   G   G   G   S

(3)( Gly4Ser )3

1 GGTGGTGGTGGTTCTGGTGGTGGTGGTTCTGGTGGTGGTGGTTCT
CCACCACCACCACGCCCACCACCACCACGCCCACCACCACCACGC
1 G G G G S G G G G S G G G G S

(4) HUMAN-IgG-Fc

1. AACAGCACGTACCGGGTTGTAAGCGTCCTCACCGTACTGCACCAGGAC
TTGTCGTGCATGGCCCAACATTCGCAGGAGTGGCATGACGTGGTCCTG
N S T Y R V V S V L T V L H Q D
49. TGGCTGAATGGCAAGGAATACAAATGCAAG
ACCGACTTACCGTTCCTTATGTTTACGTTC
W L N G K E Y K C K

(5) HSA
1. TTCCAGAATGCGCTGCTGGTTCGTTACACCAAGAAAGTACCCCAAGTGTCAACTCCAACT
AAGGTCTTACGCGACGACCAAGCAATGTGGTTCTTTCATGGGGTTCACAGTTGAGGTTGA
F G N A L L V R Y T K K V P Q V S T P T

61 CCTGTAGAGGTCTCA
GGACATCTCCAGAGT
P V E V S

(6) C-myc
1. GAACAAAAACTCATCTCAGAAGAGGATCTGAAT
CTTGTTTTTGAGTAGAGTCTTCTCCTAGACTTA
E Q K L I S E E D L N

The trispecific antibody has better been ligated to form a cyclic molecule using following interlinker peptides.

(1) HINGE (reverse):HUMAN-IgG3'CL

P C R P C T H T T D G L P T K L E

(2) HINGE (forward):HUMAN-IgG3'CL

E L K T P L G D T T H T C P R C P

The present invention provides a nucleotide sequence coding for the cyclic single-chain trispecific antibody mentioned in the invention.

In another aspects, the present invention provides an expression vector containing above mentioned nucleotide sequences. The expression vector can be pTRI.

In yet another aspects, the present invention provides a host cell containing above mentioned expression vector. The host cell can be *Escherichia coli.*

The design and construction of the trispecific antibody mentioned in this invention is based on following theory. The activation of T lymphocyte needs a co-stimulating signal. The gene coding for an antibody against human carcinoma is fused with the sequences of two reshaped antibody against two main stimulation signal molecules. The present trispecific antibody differs from other trispecific antibodies in following characteristics:
1. The trispecific antibody is a cyclic protein molecule. A hinge region of human antibody is introduced to the flanking regions of the linear trispecific antibody molecule and the antibody is circularized by hinge region sequence through disulfide bonds. The formation of a cyclic molecule reduces the interference between different antigen-binding sites in the same molecule and makes it more stable and is easier to be transported *in vivo.*
2. All three antibodies in the trispecific antibody, especially the single-domain antibody against human CD28, are small molecule antibodies. The molecular weight of the whole trispecific antibody *(84kDa)* is rather low which make it beneficial in tumor immunotherapy.
3. The antibodies against CD28 and CD3, which are in charge of the activation of T cell are both humanized reshaped antibodies with much lower immunogenecity.
4. There is a specifically designed interlinker between every two antibodies, which makes the antibody folding correctly to proper conformation and introduces many other biological functions.
5. These three antibody molecules are linked to an entire molecule, and led to an entire molecule has three different functions.
6. The anti-tumor antibody of this trispecific antibody can be replaced by other tumor-specific or cytokine-specific antibodies easily, this feature will broaden its scope of utilization.
7. It is designed to be produced by *E. coli* and the products need no more modification *in vitro.* So it is easy to be produced at low cost.

### BRIEF DESCRIPTION OF THE DRAWINGS

Fig.1 is a flow chart of construction and expression of the cyclic trispecific single- chain antibody;

Fig.2 is a illustration of the ligation of different antibodies *(anti-tumor scFv ×anti-CD3 ×anti-CD28)* and interlinkers;

Fig.3 is two DNA sequences and the putative amino acid sequences of reshaped single-domain antibody *(VH)* against CD28;

Fig.4 is the DNA and amino acid sequences of interlinkers;

Fig.5 is a view of the overlapping PCR;

Fig.6 is a physical map of universal expression vector pTRI used for cyclic single-chain trispecific antibody;

Fig.7 is a pattern of SDS-PAGE of the cyclic anti-ovarian carcinoma trispecific antibody expressed in pTRI;

Fig.8 is Western blotting results of the cyclic single-chain trispecific antibody against ovary carcinoma, which was expressed in E. coli, and the left view is Lane 1, supernatant of vector pTMF; Lane 2, supernatant of TsAb, and the right view is Lane 1, supernatant of TsAb (*400ug*/*ml*); Lane 2, supernatant of TsAb (*40ug*/*ml*); Lane 3, supernatant of TsAb (*4ug*/*ml*);

Fig.9 is ELISA results of reaction between the cyclic single-chain trispecific antibody against ovary carcinoma with antigen CD28, wherein Control was supernatant of vector pTMF; TRI was supernatant of TsAb;

Fig.10 is ELISA results of reaction between the cyclic single-chain trispecific antibody against ovary carcinoma with membrane antigen of ovary carcinoma cells or antigen CD3, wherein PTMFSKOV was a reaction between supernatant of vector pTMF with membrane antigen of ovary carcinoma cells; TRISKOV was a reaction between TsAb with membrane antigen of ovary carcinoma cells; PTMFJUR was a reaction between supernatant of vector pTMF with membrane antigen of Juekat cells; TRIJUR was a reaction between TsAb with membrane antigen of Jurkat cells;

Fig.11 is the cytotoxicity in vitro of the cyclic single-chain trispecific antibody against ovary carcinoma to ovary carcinoma cells *(OCCD3CD28: anti-ovary carcinoma scFv + anti-CD3 antibody + anti-CD28 antibody; OCCD3: anti-ovary carcinoma scFv + anti-CD3 antibody; TRI: cyclic single-chain trispecific antibody; Control: no antibodies; VectorCK: supernatant of vector); and*

Fig. 12 is a rosette formation assay of the cyclic single-chain trispecific antibody.

### DETAILED DESCRIPTION OF THE INVENTION

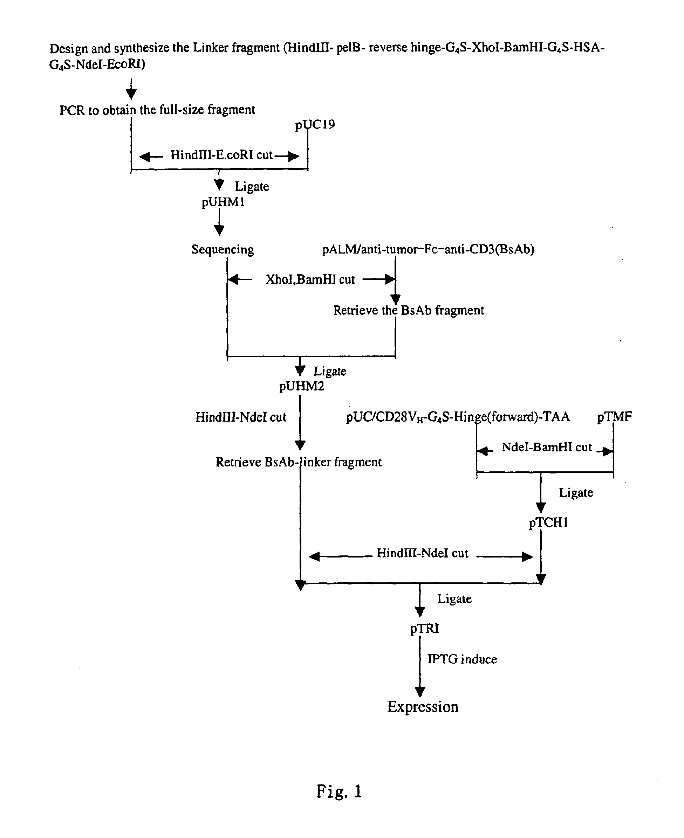

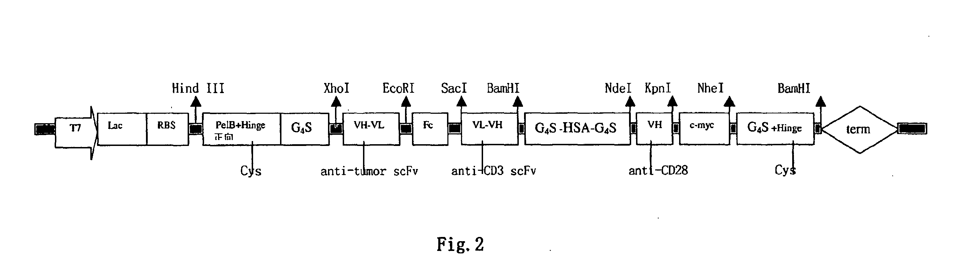

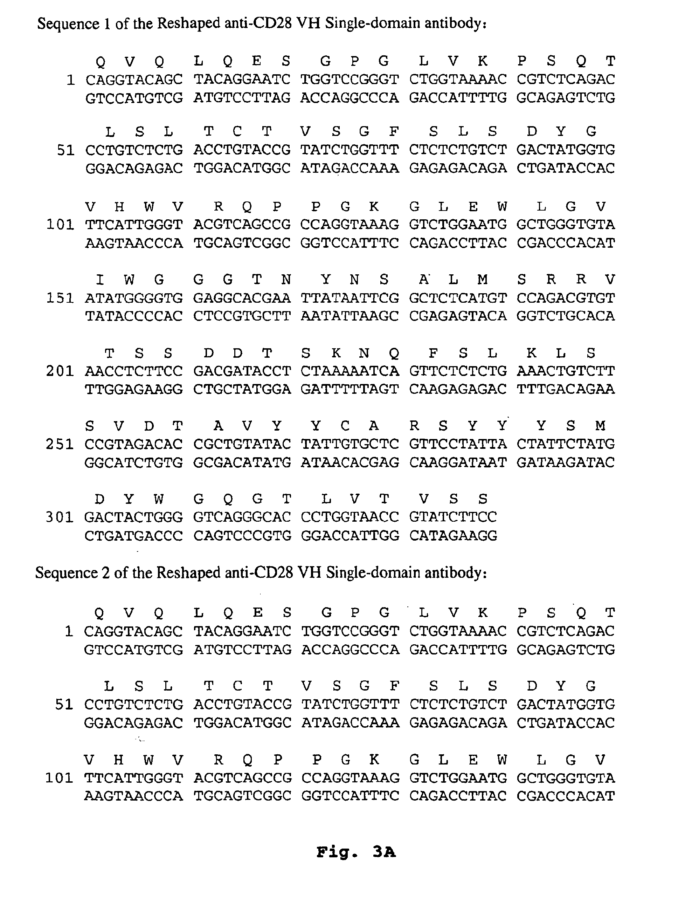

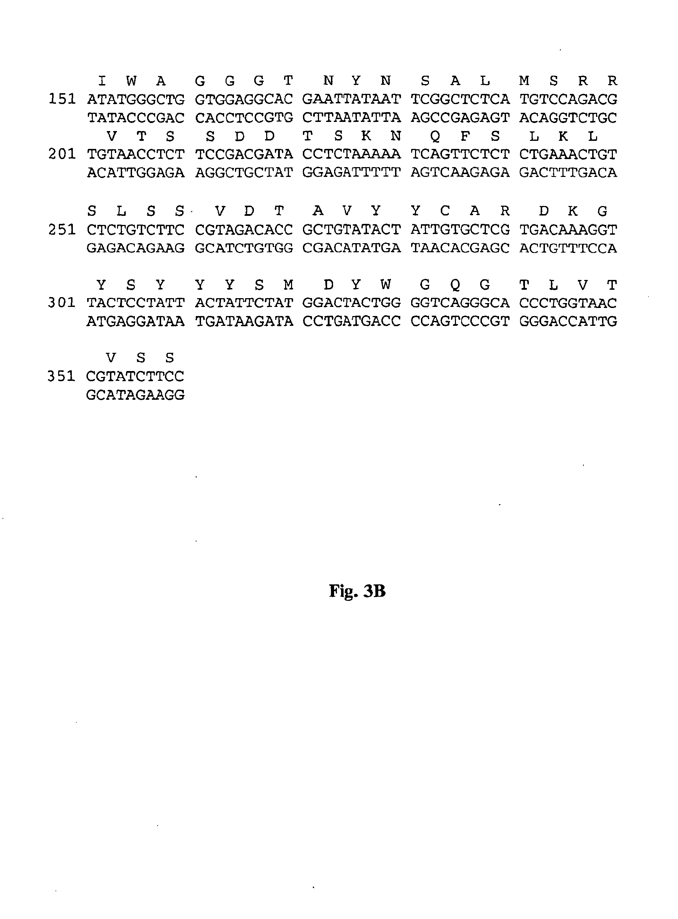

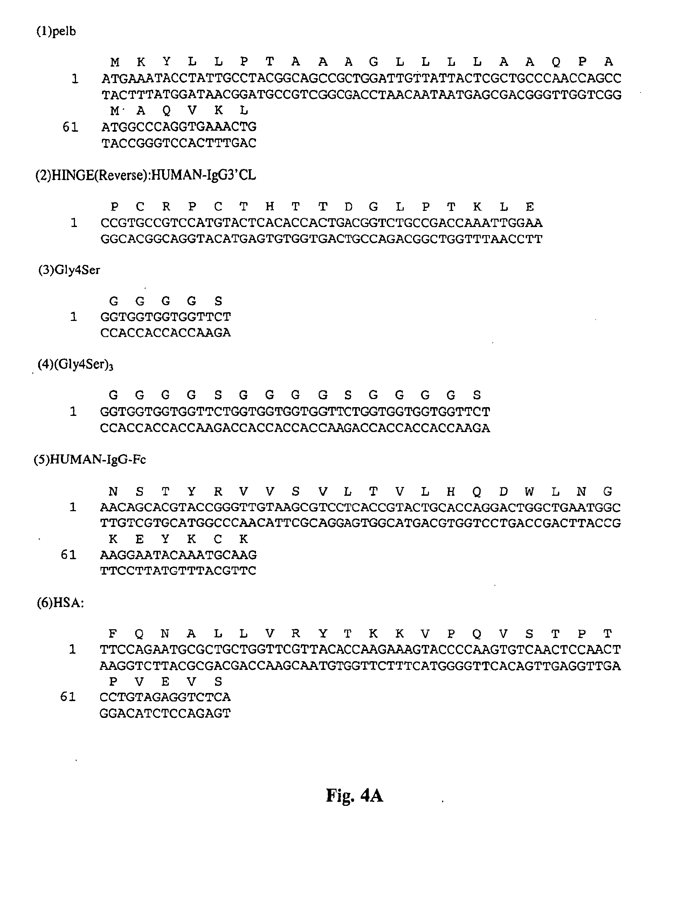

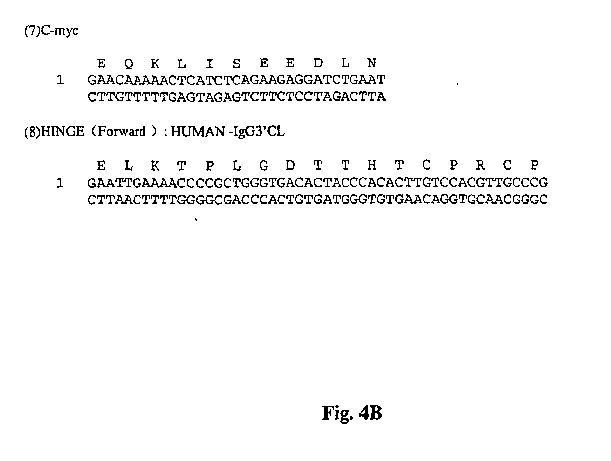

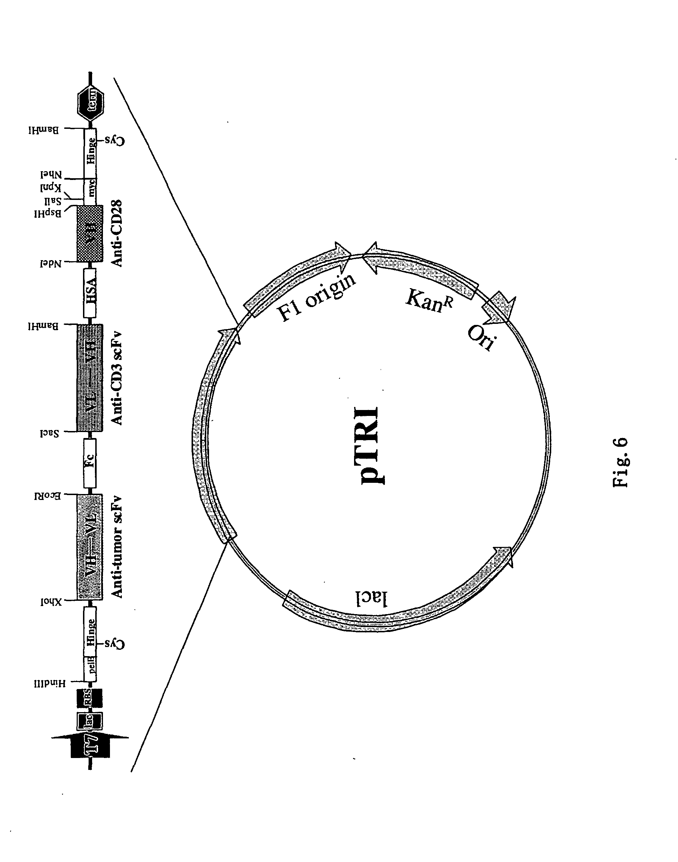

The interlinker sequence was artificially synthesized by using overlapping PCR. A new plasmid named pUHM1 was generated by insertion this interlinker sequence into pUC19. The DNA fragment of bispecific antibody against ovarian carcinoma× CD3 was achieved by digesting plasmid pALM-Fc with *Xho*I and *BamHI* and then was inserted into pUHM1. The plasmid containing this sequence is named pUHM2. Another-expression plasmid pTCH1 was generated by inserting the reshaped single-domain antibody against CD28 and interlinker into pTMF. The fragment of anti-ovarian carcinoma×anti-CD3 bispecific antibody and interlinker was digested from pUHM2, and then was inserted into pTCH1. The final expression vector, named pTRI was used to transform BL21 competent cells. The clones that had been proved to be pTRI positive were inoculated to LB medium with 50 µ g/ml Kanamycin, cultured at 37°C with vigorous shaking to OD₅₅₀ 0.4-0.5. The culture was induced with IPTG to final concentration of 0.8mmol/L for 4 hours and then harvested by centrifugation. The cells were lysed by ultrasonic and the lysate was centrifuged at 12,000 rpm for 10 minutes, the supernatant and pellet were separated on 8% and 12% SDS-PAGE. The samples were also analyzed by standards procedures, including immunoblotting, immunological activity and cytological assay (see Fig.1~Fig.6).

The protocols in detail are listed as follows.

### 1. Construction of cloning vector pUMH1

This cloning vector is derived from pUC19. A linker sequence of 5'-*Hind*III-pelB-human IgG3'CL
hinge(complementary)-Gly₄Ser-HSA-Gly₄Ser-N*de*I-*Eco*RI-3' was inserted into pUC19 linearized with *Hin*dIII and *Eco*RI.

Six oligonucleotide fragments, named P1~P3 and RE1~RE3 were used in SOE-PCR as template/primer to get a 285 bp entire linker fragment:

P1: 5'-CCCAAgCTTATgAAATACCTATTgCCTACggC-3' 32nts

P2: 5'-GCCCAGGTGAAACTGCCGTGCCGTCCATGTACTCACACCACTGACGGTCTGCCGACCAAATTGGAA
GGTGGTGGTGGTTC-3' 80nts

P3: 5'-CTGCTGGTTCGTTACACCAAGAAAGTACCCCAAGTGTCAACTCCAACTCCTGTAGAGGTCTCAGGTGG
TGGTGGTTCTCAT-3' 81nts

RE1: 5'-CCggAATTCCATATgAgAACCACCACCACC - 3' 30nts

RE2: 5'-TTCTTGGTGTAACGAACCAGCAGCGCATTCTGGAAAGAACCACCACCACCGGATCCCTCGAGAGAACC
ACCACCACCTTCC -3'    81nts

RE3: 5'-GGCACGGCAGTTTCACCTGGGCCATGGCTGGTTGGGCAGCGAGTAATAACAATCCAGCGGCTGCCGTA
GGCAATAGGTATT-3'    81nts

### 1.1 Overlapping PCR used in construction of linker sequence

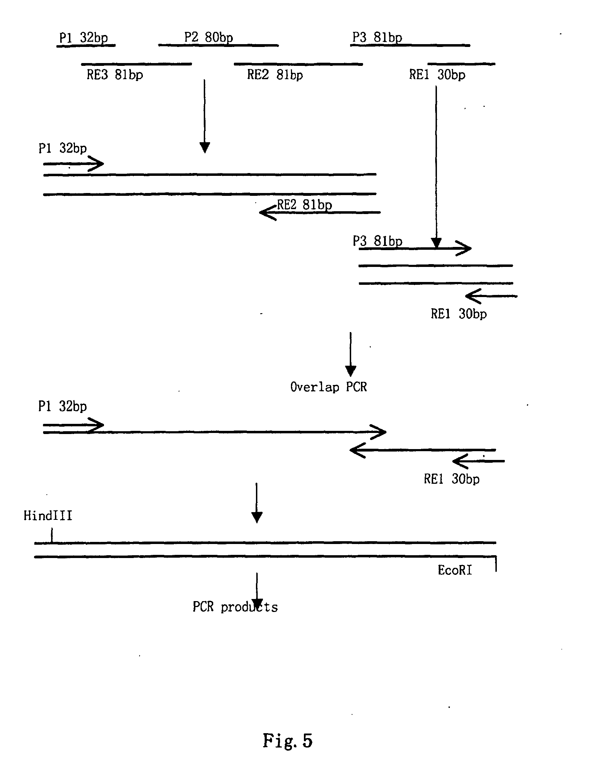

Overlapping PCR was carried out with two steps as shown in Fig.5. First step: Two double-stranded products M1 and M2 were assembled with P1, P2, RE2 and RE3, P3 and Re1, respectively. Second step: The entire linker was got by using overlapping PCR with equal molar of M1 and M2 as templates.

Generation of M1: In a 30 µl reaction, adding 4 µl (~10pmol/L) of P1, P2, RE2 and RE3, respectively, 3 µl of 10×pfu DNA polymerase buffer, 4µl of dNTPs (2mmol/L each), 1 µl of pfu DNA polymerase, adding deionized H2O to adjust total volume to be 30 µl, overlaid with 100 µl paraffin oil. Run 30 PCR cycles on a thermal cycler. The thermal cycle is 94°C for 1min, 55°C for 30 sec and 72°C for 40 sec. The amplified DNA fragments are analyzed on 2.5% agarose gel. The target band was cut out and recovered using Gel DNA purification kit (Watson Inc. Shanghai, China ).

Generation of M2: In a 30 µl reaction, adding 10 µl of P3, RE1 (*∼10pmol, each*), 3 µl of 10×PCR buffer, 4 µl of dNTPs (*2mM, each*)*,* 1 µl of *pfu* DNA polymerase , adding deionized H₂O to adjust total volume to be 30 µl, overlaid with 100 µl of paraffin oil. Run 30 PCR cycles on a thermal cycler. The thermal cycle is 94°C for 1min, 60°C for 30 sec and 72°C for 40 sec. The amplified DNA fragments are analyzed on 2.5% agarose gel. The target band was cut out and recovered using Gel DNA purification Kit *(Watson Inc. Shanghai, China* ).

Generation of the full-length linker : In a 30 µl reaction, adding 5 µl of recovered M1 and M2 as templates , 2 µl of P1 and RE1 as primers, 3 µl of 10×*pfu* Buffer, 4 µl of dNTPs *(2mM, each),* 1 µl of *pfu* DNA polymerase, adding deionized H₂O to adjust total volume to 30 µl, overlaid with paraffin oil. Run 30 PCR cycles on a thermal cycler. The thermal cycle is 94°C for 1min, 55°C for 30 sec and 72°C for 40 sec. The amplified DNA fragments are analyzed on 2.5% agarose gel. The full-length band was cut out and recovered using Gel DNA purification Kit *(Watson Inc. Shanghai, China ).*

### 1.2 Restrictive endonuclease digestion and purification of PCR products

The full-length PCR product was digested with *Hind*III and *Eco*RI at 37°C for 4 hours. The digested DNA fragments were separated on 1% agarose gel. The target band was recovered using Gel DNA purification Kit *(Watson Inc. Shanghai, China* ).

### 1.3 Minipreparation of pUC19

Inoculate a single-colony of DH5 α containing plasmid pUC19 into 5ml of LB medium containing 100 µ g/ml ampicillin. Incubate the culture overnight at 37°C with vigorous shaking. Pour 1.5 ml of the culture to an eppendorf tube. Centrifuge at 12,000rpm for 1min. remove the medium and resuspend the bacterial pellet in 100 µl of solution I (*50mmol*/*l glucose*, *10mmol*/*l EDTA, 25mmol*/*l Tris-Cl8.0*) by vigorous vortexing. Add 200 µl of freshly prepared solution *II(0.2mol*/*l NaOH, 1% SDS)* to bacterial suspension, close the tube tightly and then mix the contents by inverting the tube several times. Store the tube on ice for 3min, add 150 µl of ice-cold solution III(*3mol*/*L KAc, pH4.8*). Close the tube and invert several times softly. Store the tube on ice for 5 min. Centrifuge the bacterial lysate at 12,000rpm at 4°C for 10 min. transfer the supernatant to a fresh tube. Precipitate DNA from the supernatant by adding two volumes of ice-cold ethanol. Mix the solution by vortex and then allow the mixture to stand for 10 min. at room temperature. Centrifuge at 12,000rpm for 20 min. at 4°C. Add 75% ethanol to wash the DNA pellet once, store the opened tube at room temperature to dry. Dissolve the pellet in 100 µl H₂O containing 50µg/ml DNase-free RNaseA. Store the tube at 37°C for 30min. Add an equal volume of Phenol: chloroform(1:1). Mix the organic and aqueous phases by vortex and then centrifuge the emulsion at 12,000rpm for 2min. at room temperature. Transfer the aqueous upper layer to a fresh tube. Add an equal volume of chloroform: isopentanol (*24:1, V*/*V*). Mix the organic and aqueous phases by vortex and then centrifuge the emulsion at 12,000rpm for 2min at room temperature. Transfer the upper aqueous layer to a fresh tube. Add 1/10 volume of NaAc (*3mol*/*l, pH5.2*) and two volumes of ice-cold ethanol. Mix the solution by vortex and then allow the mixture to stand for 10 min. at room temperature. Centrifuge at 12,000rpm for 60 min. at 4°C. Add 75% ethanol to wash the DNA pellet once, store the opened tube at room temperature to dry out. Dissolve the pellet in 25 µl H₂O.

### 1.4 Restrictive endonuclease digestion and purification of plasmid pUC19

One microgram of pUC19 DNA was digested with *Hind*III and *Eco*RI in 40 µl system (*4 µl 10×buffer, 30U HindIII and 30U EcoRI*) at 37°C for 4 hours. The reaction mixture was analyzed on 1% agarose gel. The target band was recovered using Gel DNA purification Kit *(Watson Inc. Shanghai, China ).*

### 1.5 Ligation of linearized pUC19 and linker fragment

About 40 ng of recovered double-digested pUC19 fragment and about 20 ng of double-digested PCR product were used in a 20 µl ligation reaction( 2 µl 10× T₄ DNA ligase buffer, 1µl T₄ DNA ligase ). Incubate overnight at 16°C.

### 1.6Preparation of competent E.coli cell using Calcium Chloride

Pick a single colony of Top 10 from a plate. Transfer the colony into 3ml of antibiotic-free LB medium. Incubate the culture at 37°C overnight with shaking. Transfer 300 µl of the culture to 30 ml of LB medium. Incubate the culture to OD₅₅₀0.3~0.4(about 3 hours) at 37°C with vigorous shaking. Store the culture on ice for 10 min to cool down then centrifuge at 4,000 rpm for 10 min at 4°C. Remove the supernatant, add 20ml ice-cold 0.1mol/L CaCl₂ to resuspend the pellet by swirling or gentle vortex. Cool the culture by storing the tube on ice for 30 min. Centrifuge at 4,000rpm for 10min at 4°C. Remove the supernatant, add 2ml ice-cold 0.1mol/l CaCl₂ *(containing 12% glycerol)* to resuspend the pellet. Add each 200 µl of this competent cell to eppendorf tube then store at -70°C.

### 1.7 Transformation of Ecoli, Screening Bacterial Positive Colonies and Identification by sequencing

Gently mix 10µl ligation product with 200µl DH5 a chemical competent cell, keep on ice for 30 minutes, then incubate in 42°C water bath for exactly 90 second, then cool on ice for 5 minutes, transfer all of 200µl transformed competent cells onto agar LB medium containing 100 µg/ml Ampicilline. After the liquid has been absorbed, invert the plate and incubate at 37°C overnight. Pick single colony for mini-preparation of plasmid DNA by alkaline lysis method. Identify the plasmid by digestion with HindIII and EcoR I and then amplified with P1 and RE1 as primers. The positive plasmid was further identified by sequencing, designated as pUMH1.

### 2. The construction of cloning vector pUMH2

The cloning vector pUMH2 is originated from palsmid pUMH1 with insertion of a bispecific antibody fragment *(5'-XhoI- anti-ovarian carcinoma scFv -Fc linker -anti-CD3 scFv-BamHI-3'*) between *Xho*I and *Bam*HI*.* The anti-ovarian× anti-CD3 bispecific single-chain antibody fragment is digested from pALM-Fc constructed in our lab.

### 2.1 Preparation and Purification of bispecific antibody fragment

Extraction plasmid DNA of pALM-Fc by Alkaline lysis, digest 1µg of pALM-Fc with *XhoI* and *BamHI* in a 40 µ l reaction containing 30U *Xho*I and *Bam*HI (*TaKaRa, Dalian, China*) ,4 µl 10×buffer, incubate at 37°C for 4 h. The product was separated on 1% agarose gel. The target band was then cut out and recovered by using Gel purification Kit *(Waston Inc, Shanghai, China).*

### 2.2 Digestion and purification of plasmid pUMH1

Digest 1µg of pUMH1 DNA with *Xho*I and *Bam*HI in 40 µl volume as described above, incubate at 37°C for 4 h. Extract the digested fragment with Gel purification Kit ( *Waston INC, Shanghai, China).*

### 2.3 Ligation, Transformation and Screening of positive colony

Set up ligation reaction as follows:

| | |
|---|---|
| Double digested pUMH1 | 40ng |
| Double digested fragment of bispecific antibody | 20ng |
| 10×T4 ligation buffer: | 2 µL |
| T4 DNA ligase: | 2 µ L |
| Nuclease-free water to final volume | 20 µ L |

Incubate at 16°C overnight. Gently mix 10 µl ligation mixture with 200µl DH5α competent cell, place the cells on ice for 30 min, then incubate in 42°C water bath for exactly 90 second, then cool on ice for 5 min, transfer all of 200µl transformed competent cells onto agar LB medium containing 100 µg/ml ampicillin. After the liquid has been absorbed, invert the plate and incubate at 37°C overnight. Pick single colony for mini-preparation of plasmid DNA. Identify the insert fragment with *Xho*I, *Bam*HI; *Hind*III and *Eco*RI. The identified positive plasmid was pUMH2.

### 3. Construction and expression of cyclic single-chain trispecific antibody

The construction of the cyclic single-chain trispecific antibody was based on the expression vector pTCH1 and bispecific fragment from pUMH2. pTCH1 was derived from vector pTMF containing -*Nde*I-( VH of anti-CD28 scFv)-(c-myc)-Gly4Ser-Human IgG3'CL(17aa, Forward)-*Bam*HI.

### 3.1 Construction of expression plasmid pTCH1

Extract the recombinant plasmid pUC19 containing-NdeI-(anti-CD28 VH)-(c-myc)-Gly₄Ser-Human IgG3'CL *(17AA, forward*)-BamHI- fragment by alkaline lysis mini-preparation. Then, Digest 1µg of the plasmid DNA with NdeI and BamHI in a 40 µl system(*4 µL* 10*×buffer, 30U NdeI, BamHI each*), incubate in a water bath at 37°C for 4 h. Digest the vector pTMF at the same time. The product was separated on 1% agarose gel. The target band was then cut out and recovered by using Gel purification Kit *(Waston Inc, Shanghai, China).* Set up ligation reaction:

| | |
|---|---|
| Double digested pTMF | 40ng |
| Double digested fragment of anti-CD28 scFv | 20 ng |
| 10×T4 ligation buffer: | 2 µL |
| T4 DNA ligase: | 2 µL |
| Nuclease-Free Water to final volume | 20 µ L |

Incubate at 16°C overnight.

Gently mix 10 µl ligation mixture with 200 µl chemical competent BL21 cell, place the cells on ice for 30 min, then incubate in 42°C water bath for exactly 90 sec, then cool on ice for 5 min, transfer all of 200 µl transformed competent BL21 cells onto agar LB medium containing 50µg/ml kanamycin. After the liquid has been absorbed, invert the plate and incubate at 37°C overnight. Pick single colony for mini-preparation of plasmid DNA. Identify the insert fragment with *Nde*I and *Hind*III digestion and the size of plasmid. The positive plasmid was named as pTCH1.

### 3.2 Restrictive endonuclease digestion and Purification of vector plasmid pTCH1

Digest 1µg of pTCH1 DNA with *Nde*I and *Hind*III in a 40µl system*(4 µ L 10×* buffer, *30U NdeI and HindIII*)*,* incubate at 37°C for 4 h. The product was separated on 1% agarose gel. The target band was then cut out and recovered by using Gel purification Kit *(Waston Inc, Shanghai, China).*

### 3.3 Preparation and purification of interlinker containing bispecific antibody

Mini-preparation of plasmid pUMH2 with alkaline lysis method. Digest 1µg of pUMH2 DNA with *Nde*I and *Hind*III in a 40µl system (*4 µL 10*× *buffer, 30U NdeI and HindIII),* incubate at 37°C for 4 h. The product was separated on 1% agarose gel. The target band was cut out and recovered by using Gel purification Kit *(Waston Inc, Shanghai, China)*

### 3.4 Ligation, Transformation and Screening of positive colony

Set up ligation reaction:

| | |
|---|---|
| Double digested pTCH1 | 40ng |
| Double digested bispecific fragment | 20ng |
| 10×T4 ligation buffer | 2 µL |
| T4 DNA ligase | 2 µL |

Add H₂O to final volume 20 µL

Incubate at 16°C overnight. Gently Mix 10µl of ligation product with 200µl BL21 chemical competent cells and keep on ice for 30 minutes, incubate in 42°C water bath for exactly 90 sec, then cool on ice for 5 min, transfer all of 200µl transformed competent cells onto agar LB medium containing 50µg/ml kanamycin. After the liquid has been absorbed, invert the plate and incubate at 37°C overnight. Pick single colony for mini-preparation of plasmid DNA. Identify sample with *Nde*I and *Hin*dIII. The positive plasmid was named as pTR1.

### 3.5 Expression of cyclic single-chain trispecific antibody

Pick a fresh pTR1 positive single colony into LB media containing 50µg/ml Kanamycin, incubate at 37°C overnight. Dilute the overnight cultures 1:100 in 50ml of fresh LB medium in a 250ml flask. Incubate the culture to grow at 37°C until the cells reach mid-log growth *(OD*_{*600*} *0.4~0.5),* add IPTG to the culture to a final concentration of 0.8mM, incubate the culture for a further 4h. Collect and sonicate the bacteria on ice, 12000 rpm centrifuge for 10 minute, analyze the supernatant and the pellet by 8%, 12% SDS-PAGE.

| **Casting of SDS-polyacrylamide gel** | | | | |
|---|---|---|---|---|
| | Stacking gel 5% | Separating gel 12% | Separating gel 8% | Sealing gel |
| 30% Acrylamide stock: | 0.5 | 6.0 | 4.0 | 0.53 |
| (29:1)(ml) | | | | |
| H₂O(ml): | 2.1 | 4.9 | 6.9 | 0.93 |
| 1 M Tris-HCl: | 0.38 | - | - | 0.05 |
| (pH 8.8)(ml) | | | | |
| 1.5 M Tris-HCl: | - | 3.8 | 3.8 | - |
| (pH 6.8) (ml) | | | | |
| 10% SDS(ml): | 0.03 | 0.15 | 0.15 | 0.02 |
| 10% Peroxydisulphate (ml): | 0.03 | 0.15 | 0.15 | 0.02 |
| TEMED (ml): | 0.003 | 0.006 | 0.009 | 0.0012 |
| * 29:1 w:w ratio of acrylamide to N,N'-methylene bis-acrylamide | | | | |

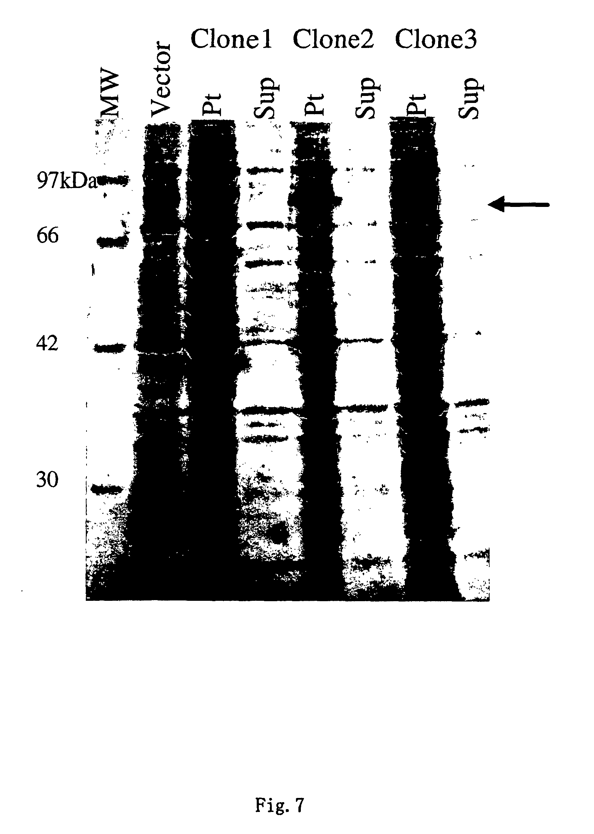

Sample preparation: Take protein sample and mix with equal volume of loading buffer(*100mM Tris-HCl pH 6.8, 200mM DTT, 4% SDS 20% glycerol, 0.2% bromophenol blue*), heat e at 100°C for 5 min prior to load each sample onto an SDS-polyacrylamide gel Electrophoresis: Run the gel at 60V in stacking gel, then 120V in separating gel in electrophoresis buffer *( 25mM Tris, 0.1% SDS, 250mM Glycine (pH8.3) ).* Stain proteins in the gel for 1 to 2 hr in Coomassie blue R-250 staining solution (0.25% (w/v) Coomassie Brilliant Blue R 250, 50% methanol, 10% acetic acid). Follow by destaining with 10% acetic acid *(50% methanol,* 10% acetic acid), changing the solution every 30 min until background is clear (3 to 5 changes). Take pictures and analyze the gels ( *shown in Fig. 7).*

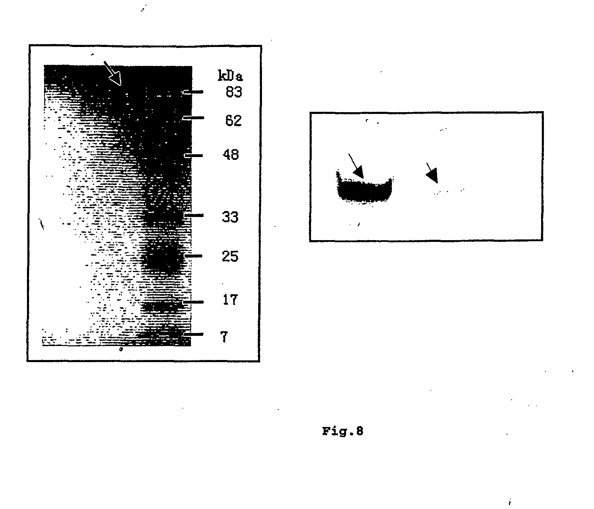

Western-blot: the Protein samples are transferred from polyacrylamide gels to PVDF membrane by electrophoresis *(Bio-Rad mighty small transphor system).* Electrophoresis blotting is performed according to the protocol provided by manufacturer. Briefly, incubate the membrane in the blocking solution *(5% skimmed milk)* for two hours and wash it in TBST three times for 5 minutes each. Transfer the membrane to TBST containing 1:1,000 dilution of mouse anti-c-myc IgG and incubate for 1 hour at room temperature. Wash the membrane in TBST three times for 5 minutes each. Transfer the membrane to TBST containing goat anti-mouse IgG HRP conjugate *(1:1000 dilution)* and incubate for 1 hour at room temperature. Wash the membrane in TBST five times for 5 minutes each. At last, incubate the membrane in substrate solution (*6mg*/*ml DAB, 1% H*_{*2*}*O*_{*2*}) until the bands of interest have reached the desired intensity. Stop the reaction by washing the membrane in deionized water for several times.. The molecular weight of scTsAb is 84kDa *(shown in Fig. 8).*

### 4. Functional characterization of cyclic single-chain trispecific antibody in vitro

### 4.1 The antigen binding activities of cyclic single-chain trispecific antibody

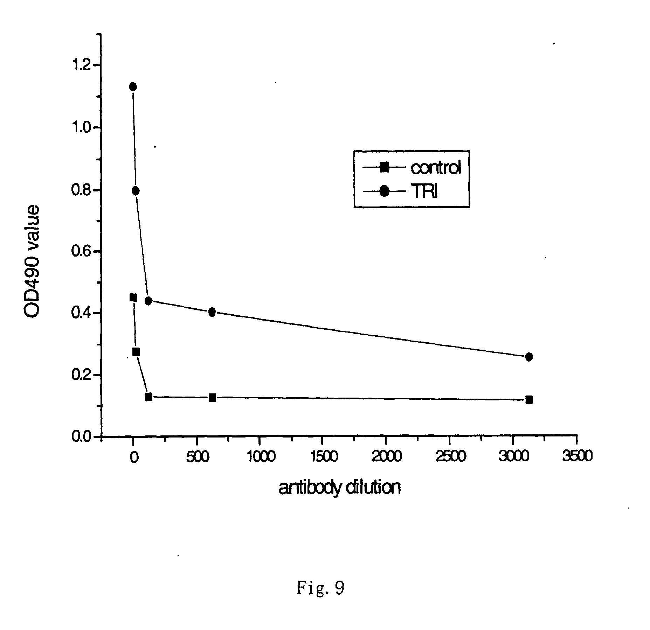

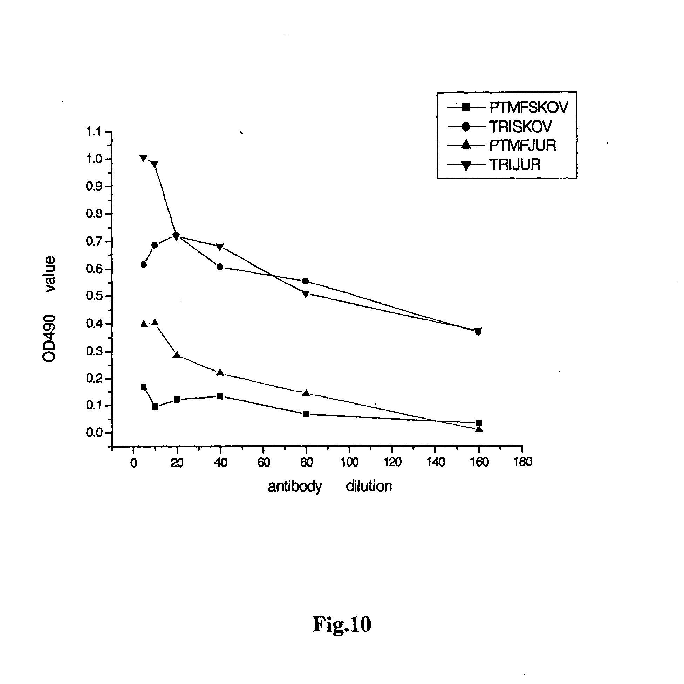

The antigen binding activities of sTRI to rhCD28/Fc antigen and cell membrane antigen of Jurkat cell and SKOV-3 cell are studied by enzyme-linked immunosorbant assay *(ELISA).* Briefly, cell membrane antigen is prepared with ultrasonic disruption of tumor cells. ELISA was performed with the antigen immobilized on 96-well plates. Mouse anti-c-myc antibody *(9E10)* is used as primary antibody and HRP-conjugated goat anti-mouse IgG as secondary antibody. At last, visualize the result with OPD as substrate and measure the absorbance at 490 nm. As shown in Fig 9 and Fig 10, the cyclic single-chain trispecific antibody can bind to three kinds of antigen specifically.

### 4.2 In vitro cytotoxity assay

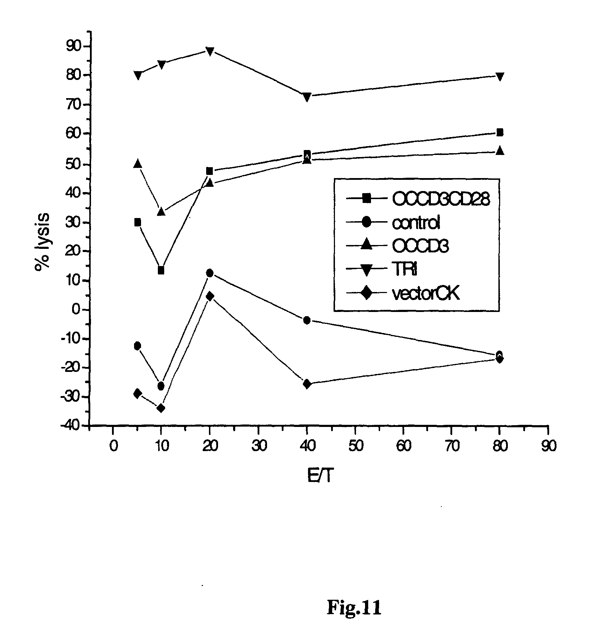

Human peripheral blood lymphocytes *(PBL)* of healthy donors are obtained by Ficoll gradient separation, monocyte/macrophage fraction is depleted by glass adherence method(*37°C 2 hours).* SKOV-3 cells are plated in flat-bottom 96-well plate to prepare cell monolayer. Freshly isolated effector cells (*PBL*) were added to the monolayer of tumor cells at appropriate ratios with different dilutions of supernatant containing sTRI at the same time and incubate overnight at 37°C for 3 days in 5% CO₂ Incubators. Wash the plate two times with RPMI 1640 medium to remove effector cells. Add 200ul RPMI 1640 medium and 20µl MTT solution (*0.5mg*/*ml, sigma*) and incubate at 37°C for 4 hours. After discarding the MTT supernatant, add 100ul DMSO to dissolve the formazan and read the sample at OD₅₇₀. Prepare the blank wells by adding medium only and the control wells by adding target cells and effector cells. Design three replicates for each sample. The percent of cytotoxicity is calculated as the following formula: Percent cytotoxicity *=(absorbance of control wells - absorbance of experiment wells* / *absorbance of control wells absorbance of blank wells)* × 100. As shown in Fig. 11, adding of the cyclic single-chain trispecific antibody results specifically killing effects to tumor cells. Percent of cytotoxicity of the cyclic single-chain trispecific antibody group is obviously higher than the other two experiment sets *(oc-scFv+CD3scFv and oc-scFv+CD3 scFv+CD28 scFv)*.

### 4.3 Rosette formation assay

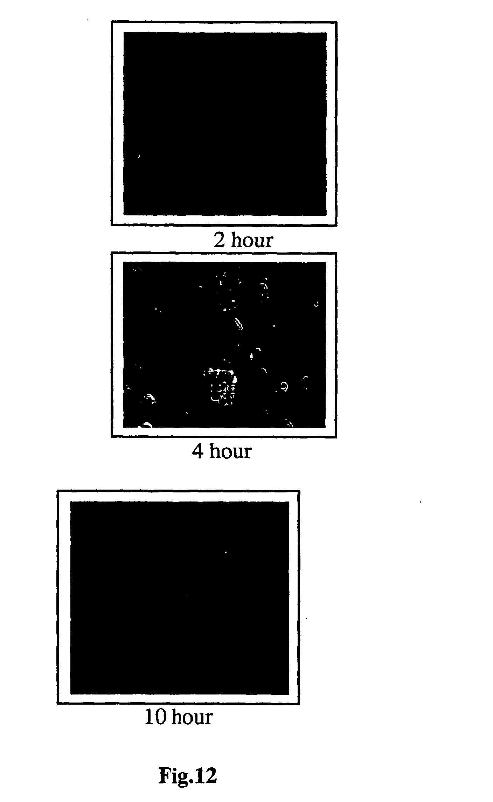

Centrifugate the trypsinized SKOV-3 cells for 5 minutes at 1,000 rpm, discard the supernatant and resuspend the cells in complete medium *(10% bovine serum, RPMI 1640* ). Add 2×10² SKOV-3 cells per well and incubate at 37 °C overnight in 5% CO₂ incubator. PBLs isolated as above are activated by adding 100 IU IL-2 at 37°C overnight at the same time. PBLs are washed three times with RPMI 1640 to remove residual IL-2 and added into SKOV-3 plate with E/T ratio of 20:1*(E: effector cells, PBLs; T: target cells, SKOV-3 cells*). Meanwhile, cyclic single-chain trispecific antibody supernatant is supplemented. Incubate the mix above at 37°C in 5% CO₂ incubator and photograph under inverted microscope at the intervals of two hours. As shown in Fig. 12 and Table 2, after two hours the effector cells begin to adhere with the target cells. After four hours the target cells started to break. At last, after 10 hours, most of target cells fall to pieces.

| Table 2. | | | | | |
|---|---|---|---|---|---|
| Percent of Rosette formation mediated by the cyclic single-chain trispecific antibody | | | | | |

| Concentration of sample | 400µg/ml of antibody | 40µg/ml of antibody | 4uµg/ml of antibody | 40µg/ml of vector supernatant | Blank |
|---|---|---|---|---|---|
| Percent of wreath formation | 40% | 30% | 20% | 10% | 0 |

## Claims

1. A cyclic single-chain trispecific antibody against human tumor, comprising three parts connected together, a first part thereof having an anti-tumor Fab antibody ,an anti-tumor single-domain antibody or an scFv; a second part thereof having a reshaped Fab antibody against human CD3, a reshaped single-domain antibody against human CD3 or a reshaped scFv against human CD3, and a third part thereof having a reshaped Fab antibody against human CD28, a reshaped single-domain antibody against human CD28 or a reshaped scFv against human CD28.

2. The cyclic single-chain trispecific antibody as claimed in claim 1, wherein the anti-tumor Fab antibody, single-domain antibody or scFvis Fab antibody against human ovarian carcinoma, single-domain antibody against human ovarian carcinoma or scFv against human ovarian carcinoma respectively.

3. The cyclic single-chain trispecific antibody as claimed in claim 1, wherein it comprises an anti-tumor scFv, a reshaped scFv against human CD3 and a reshaped single-domain antibody against human CD28.

4. The cyclic single-chain trispecific antibody as claimed in claim 3, wherein the reshaped single-domain antibody against human CD28 is a reshaped V_{H} fragment with one of following two amino acid sequences: 
QVQLQESGPGLVKPSQTLSLTCTVSGFSLSDYGVHWVRQ
PPGKGLEWLGVIWGGGTNYNSALMSRRVTSSDDTSKNQ
FSLKLSSVDTAVYYCARSYYYSMDYWGQGTLVTVSS
(113aa)
 or

Q V Q L Q E S G P G L V K P S Q T L S L T C T V S G F S L S D Y G V H W V R Q
P P G K G L E W L G V I W A G G G T N Y N S A L M S R R V T S S D D T S K N Q
F S L K L S L S S V D T A V Y Y C A R D K G Y S Y Y Y S M D Y W G Q G T L V T V S S
(126aa)

5. The cyclic single-chain trispecific antibody as claimed in claim 1, wherein there are some kinds of linker peptide between the anti-tumor Fab, anti-tumor single-domain antibody or anti-tumor scFv, and the reshaped Fab, reshaped single-domain antibody or reshaped scFv against human CD3, and the reshaped Fab, reshaped single-domain antibody or reshaped scFv against human CD28.

6. The cyclic single-chain trispecific antibody as claimed in claim 5, wherein the linker peptides are one of following six amino acid sequences:

(1) MKYLLPTAAAGLLLLAAQPAMAQVKL

(2) GGGGS

(3) GGGGSGGGGSGGGGS

(4) NSTYRVVSVLTVLHQDWLNGKEYKCK

(5) FQNALLVRYTKKVPQVSTPTPVEVS

(6) EQKLISEEDLN

7. The cyclic single-chain trispecific antibody as claimed in claim 5, wherein the antibody is linked to a cyclic molecule by using following two interlinker peptides: PCRPCTHTTDGLPTKLE or

ELKTPLGDTTHTCPRCP

8. A polynucleotide sequence coding for the cyclic single-chain trispecific antibody mentioned in any of claim 1 to 7.

9. An expression vector containing nucleotide sequences as claimed in claim 8.

10. The expression vector according to claim 8, wherein it is pTRI.

11. A host cell containing the expression vector mentioned in any of claim8 or claim 9.

12. The host cell according to claim 10, wherein it is *Escherichia coli*.

13. A drug complex for therapy or prevention of cancer, comprising the cyclic single-chain trispecific antibody as any one of claim 1 to claim 7 and pharmic vector.

14. The drug complex as claimed in claim 13 wherein the drug complex is used for therapy or prevention of ovary carcinoma.

15. A use of the cyclic single-chain trispecific antibody as any one of claim 1 to claim 7 for preparing drugs to treat or prevent cancer.

16. The use as claimed in claim 15, wherein the cancer is ovary carcinoma.

17. A use of the cyclic single-chain trispecific antibody as any one of claim 1 to claim 7 for treating or preventing cancer.

18. The use as claimed in claim 17, wherein the cancer is ovary carcinoma.


SEQUENCE LISTING

<110>  INSTITUTE OF GENETICS,CAS
       BEIJING ABT GENETIC ENGINEERING TECHNOLOGY CO., LTD

<120>  CYCLIC SINGLE-CHAIN TRISPECIFIC ANTIBODY

<130>  IP02008

<160>  10

<170>  PatentIn version 3.1

<210>  1
<211>  113
<212>  PRT
<213>  Artificial

<400>. 1

Gln Val Gln Leu Gln Glu Ser Gly Pro Gly Leu Val Lys Pro Ser Gln
1               5                   10                  15

Thr Leu Ser Leu Thr Cys Thr Val Ser Gly Phe Ser Leu Ser Asp Tyr
            20                  25                  30

Gly Val His Trp Val Arg Gln Pro Pro Gly Lys Gly Leu Glu Trp Leu
        35                  40                  45

Gly Val Ile Trp Gly Gly Gly Thr Asn Tyr Asn Ser Ala Leu Met Ser
    50                  55                  60

Arg Arg Val Thr Ser Ser Asp Asp Thr Ser Lys Asn Gln Phe Ser Leu
65                  70                  75                  80

Lys Leu Ser Ser Val Asp Thr Ala Val Tyr Tyr Cys Ala Arg Ser Tyr
                85                  90                  95

Tyr Tyr Ser Met Asp Tyr Trp Gly Gln Gly Thr Leu Val Thr Val Ser
            100                 105                 110

Ser

<210>  2
<211>  120
<212>  PRT
<213>  Artificial

<400>  2

Gln Val Gln Leu Gln Glu Ser Gly Pro Gly Leu Val Lys Pro Ser Gln
1               5                   10                  15

Thr Leu Ser Leu Thr Cys Thr Val Ser Gly Phe Ser Leu Ser Asp Tyr
            20                  25                  30

Gly Val His Trp Val Arg Gln Pro Pro Gly Lys Gly Leu Glu Trp Leu
        35                  40                  45

Gly Val Ile Trp Ala Gly Gly Gly Thr Asn Tyr Asn Ser Ala Leu Met
    50                  55                  60

Ser Arg Arg Val Thr Ser Ser Asp Asp Thr Ser Lys Asn Gln Phe Ser
65                  70                  75                  80

Leu Lys Leu Ser Leu Ser Ser Val Asp Thr Ala Val Tyr Tyr Cys Ala
                85                  90                  95

Arg Asp Lys Gly Tyr Ser Tyr Tyr Tyr Ser Met Asp Tyr Trp Gly Gln
            100                 105                 110

Gly Thr Leu Val Thr Val Ser Ser
        115                 120

<210>  3
<211>  26
<212>  PRT
<213>  Artificial

<400>  3

Met Lys Tyr Leu Leu Pro Thr Ala Ala Ala Gly Leu Leu Leu Leu Ala
1               5                   10                  15

Ala Gln Pro Ala Met Ala Gln Val Lys Leu
            20                  25

<210>  4
<211>  5
<212>  PRT
<213>  Artificial

<400>  4

Gly Gly Gly Gly Ser
1               5

<210>  5
<211>  15
<212>  PRT
<213>  Artificial

<400>  5

Gly Gly Gly Gly Ser Gly Gly Gly Gly Ser Gly Gly Gly Gly Ser
1               5                   10                  15

<210>  6
<211>  26
<212>  PRT
<213>  Artificial

<400>  6

Asn Ser Thr Tyr Arg Val Val Ser Val Leu Thr Val Leu His Gln Asp
1               5                   10                  15

Trp Leu Asn Gly Lys Glu Tyr Lys Cys Lys
            20                  25

<210>  7
<211>  25
<212>  PRT
<213>  Artificial

<400> 7

Phe Gln Asn Ala Leu Leu Val Arg Tyr Thr Lys Lys Val Pro Gln Val
1               5                   10                  15

Ser Thr Pro Thr Pro Val Glu Val Ser
            20                  25

<210> 8
<211> 11
<212> PRT
<213> Artificial

<400> 8

Glu Gln Lys Leu Ile Ser Glu Glu Asp Leu Asn
1               5                   10

<210> 9
<211> 17
<212> PRT
<213> Artificial

<400> 9

Pro Cys Arg Pro Cys Thr His Thr Thr Asp Gly Leu Pro Thr Lys Leu
1               5                   10                  15

Glu

<210> 10
<211> 17
<212> PRT
<213> Artificial

<400> 10

Glu Leu Lys Thr Pro Leu Gly Asp Thr Thr His Thr Cys Pro Arg Cys
1               5                   10                  15

Pro